# EUROPEAN PATENT SPECIFICATION

(11) **EP 2 557 158 B1**
(45) Date of publication and mention of the grant of the patent: **31.08.2016**
(21) Application number: 11762135.9
(22) Date of filing: 23.02.2011
(51) Int. Cl.: C12Q 1/68, G01N 21/64, G01N 33/53, G01N 33/487

(54) **BIOPOLYMER ANALYSIS METHOD AND BIOPOLYMER ANALYZER**
BIOPOLYMER-ANALYSEVERFAHREN UND BIOPOLYMER-ANALYSEGERÄT
MÉTHODE D'ANALYSE DE BIOPOLYMÈRES ET ANALYSEUR DE BIOPOLYMÈRES

(30) Priority: 31.03.2010 JP 2010080098
(43) Date of publication of application: 13.02.2013
(73) Proprietor: Hitachi High-Technologies Corporation, Tokyo 105-8717 (JP)
(72) Inventor: AKAHORI, Rena, Chiyoda-ku Tokyo 100-8220 (JP); ANAZAWA, Takashi, Chiyoda-ku Tokyo 100-8220 (JP); OZAWA, Satoshi, Chiyoda-ku Tokyo 100-8220 (JP); YAZAWA, Yoshiaki, Chiyoda-ku Tokyo 100-8880 (JP); SAKAI, Tomoyuki, Chiyoda-ku Tokyo 100-8220 (JP); NODA, Hideyuki, Chiyoda-ku Tokyo 100-8220 (JP)
(74) Representative: Strehl Schübel-Hopf & Partner
(86) International application number: PCT/JP2011/000997
(87) International publication number: WO 2011/121881

(56) References cited:
- WO-A1-2009/022152
- WO-A2-2008/071982
- JP-A- 2007 170 957
- US-A1- 2009 283 412
- TROPINI, C. ET AL.: 'Multi-nanopore force spectroscopy for DNA analysis.' BIOPHYS. J. vol. 92, no. 5, March 2007, pages 1632 - 1637
- PENG, H. ET AL.: 'Reverse DNA translocation through a solid-state nanopore by magnetic tweezers.' NANOTECHNOLOGY vol. 20, no. 18, 06 May 2009, pages 185101-1 - 185101-8

## Description

### Technical Field

The present invention relates to an apparatus and a method for analyzing or otherwise determining the sequence of a nucleic acid, such as a DNA and an RNA, by using a thin membrane through which nanometer-sized pores are provided, and particularly to a technology for improving the efficiency of preprocessing by using arrayed nanopores, that is, multiple nanopores.

### Background Art

Nanopore-based approaches have received attention as a successor of a next-generation DNA sequencer. Among them, studies have been intensively conducted on a DNA analysis method using a solid-state nanopore-containing thin membrane having nanometer-sized pores (hereinafter referred to as nanopores or pores) formed by processing a semi-conductive material in a nano-fabrication process (Non Patent Literature 1). Two types of DNA analysis method using a thin membrane with pores of this type have been proposed so far. A first method is based on a blockage current. For example, a liquid container is provided on each side of a thin membrane with pores, and an electrode is provided in each of the liquid containers, which are filled with an electrolyte solution. A voltage is then so applied between the electrodes that an ion current flows through the nanopores. The magnitude of the ion current is proportional as a first-order approximation to the cross-sectional area of the nanopores. When a DNA passes through a nanopore, the DMA blocks the nanopore, resulting in a decrease in the effective cross-sectional area and hence a decrease in the ion current. The current is called a blockage current. Based on the magnitude of the blockage current, whether the DNA is a single-stranded DNA or a double-stranded DNA and the type of base are determined. A second method is based on a tunneling current and includes the steps of providing a pair of electrodes facing each other on the inner side surface or any other suitable place of each of the nanopores, applying a voltage between the electrodes, and measuring a tunneling current between a DNA passing through the nanopore and a probe. The type of base is determined based on the magnitude of the tunneling current. The type of base is similarly determined by applying an alternate current voltage between the electrodes, measuring the static capacitance between the electrodes, and detecting any change in the static capacitance when a DNA passes through a nanopore.

Large Brownian motion of a DNA that passes through a nanopore, the measurement speed of a detector not being fast enough to detect the DNA passing through the nanopore, and other problems, however, make it difficult to ensure measurement precision necessary to determine the type of each base. To control the Brownian motion of a DNA and the speed at which the DNA passes through a nanopore, several measurement approaches in which the motion of the DNA is controlled by trapping one terminal of the DNA (hereinafter referred to as trap measurement method) have been proposed. Exemplary methods of this type include a method for trapping a DNA with a cantilever used with an AFM or any other similar microscope (Patent Literature 1), a method for optically trapping a DNA bonded to a bead (Non Patent Literature 2), and a method for trapping a DNA in a potential well produced by a preset electric field (Patent Literature 2).

It is typical in related art to use a single thin membrane having one nanopore, i.e., a single nanopore, provided therein. On the other hand, it is conceivable that using a single thin membrane having a plurality of nanopores, i.e., multiple nanopores, provided therein can improve measurement efficiency per thin membrane. A method for performing intermolecular spectroscopy on a plurality of DNAs in a synchronous manner by using multiple nanopores combined with a method similar to the trap measurement method has been reported (Non Patent Literature 3, which is hereinafter referred to as related art example). In the related art example, copies of the same sample undergo multiple measurements using multiple nanopores, which allow resultant statistical values to be obtained at once. The related art example further discloses a procedure in which before the measurements start, a probe is trapped in a nanopore and the trapped probe is tracked based on a blockage current.

### Citation List

### Patent Literature

Patent Literature 1: United States Patent Publication No. 2006-0057585
Patent Literature 2: United States Patent Publication No. 2004-0144658

### Non Patent Literature

Non Patent Literature 1: Branton, D. et al., Nat. Biotech. 26, 1146-1153 (2008)
Non Patent Literature 2: Keyser, U. F. et al., Nat. Phys. 2, 473 (2006)
Non Patent Literature 3: Tropini, C. et al., Biophysical J. 92, 1632-1637 (2007)

WO 2009/022152 A1 discloses single-molecule spectroscopy apparatus including a barrier with a pore for trapping a molecule and optical sensing means for monitoring the passage of the molecule through the pore. WO 2008/071982 A2 discloses an electrode system for analysing molecules trapped in nanopores using a tunneling current.

### Summary of Invention

### Technical Problem

To improve the measurement efficiency based on the technology of the related art, the following problem is expected: To improve the measurement efficiency per thin membrane with a pore by using multiple nanopores, it is necessary for the individual nanopores to undergo measurement independently of each other. In the related art example, however, it is intended that copies of the same sample undergo multiple measurements and results are averaged. In this case, the measurements made on the nanopores basically provide the same result (are not made independently). The measurement efficiency per thin membrane with a unit pore in the related art example is the same as that provided by a single pore, and the related art example therefore has a limitation in terms of improvement in measurement efficiency because using multiple pores does not contribute to improvement in measurement efficiency per thin membrane with pores.

To improve the measurement efficiency in a modified related art example, the following problem is still expected: That is, assuming that the related art example is so modified that independent measurement is made on individual nanopores, the following nanopores coexist in a preparation stage: nanopores in which a probe or an analyte is trapped and nanopores in which no probe or analyte is trapped. That is, nanopores that are ready for measurement and nanopores that are not coexist. When measurement is then made on all the nanopores independently of each other, measurement is successfully made on the former nanopores, whereas measurement made on the latter nanopores fails, resulting in problems of not only wasted time but also unnecessarily prolonged measurement period during which an attempt to measure a sample that does not exist is repeatedly made.

The related art example therefore has problems of no improvement in the measurement efficiency per thin membrane with a unit pore even when multiple pores are used; low operation efficiency and time efficiency of a measuring apparatus even in a modified related art example; high running costs that increase with the operation period; and a long waiting period to obtain a result.

Another problem with the related art example is that multiple pores are unlikely to behave in the exactly same manner. In particular, when analyzed regions have short lengths, slight differences in behavior among the pores have a significant effect. In this case, results vary, and when the behaviors of DNAs in multiple pores are combined for evaluation in terms of dispersion, the dispersion values are likely to vary. As a result, there are problems of little information and a difficulty in obtaining precise results even when the results are averaged.

### Solution to Problem

The present invention is defined in the independent claims. Further advantageous features are set out in the dependent claims.

In an aspect, the present invention that achieved the object described above relates to a biopolymer analysis method using a first thin membrane with multiple nanopores. The method comprises a trap step of trapping a plurality of pieces of a biological polymer in the multiple nanopores, a trap information acquisition step of acquiring trap information on the plurality of pieces of the biological polymer, and an analysis step of analyzing characteristics of the biological polymer based on the trap information, as defined in claim 1.

As another aspect, the present invention relates to a biopolymer analyzer comprising a first thin membrane with multiple nanopores, trap information acquisition means for acquiring trap information on a biological polymer on the first thin membrane, and biological polymer characteristic analysis means, as defined in claim 9.

### Advantageous Effects of Invention

According to the aspects described above, no unnecessary time is spent to measure pores having trapped no DNA, unlike the waste in the related art example. The following advantageous effects are therefore provided as compared with the related art example: An expensive measuring apparatus can be operated at increased efficiency, resulting in improved temporal efficiency, that is, improved throughput; shortened operation time; reduced running cost; and shortened waiting period.

### Brief Description of the Drawing.

[Figure 1] Figure 1 is a flowchart showing an example of a biomolecular characteristic analysis process.
[Figure 2] Figure 2 is a flowchart showing another example of the biomolecular characteristic analysis process.
[Figure 3] Figure 3 is a flowchart showing another example of the biomolecular characteristic analysis process.
[Figure 4] Figure 4 shows an example of a biological polymer to which a labeling substance is bonded.
[Figure 5] Figure 5 shows that a biological polymer to which a labeling substance is bonded is trapped by a thin membrane with pores in a chamber to which a voltage is applied.
[Figure 6]Figure 6 shows how a biological polymer to which a labeling substance is bonded is trapped by a thin membrane with pores.
[Figure 7] Figure 7 shows an example of a thin membrane having multiple nanopores.
[Figure 8] Figure 8 shows an example of the arrangement of a plurality of thin membranes each having a single nanopore.
[Figure 9] Figure 9 shows an example of the arrangement of a plurality of thin membranes each having multiple nanopores.
[Figure 10(A)] Figure 10(A) shows an example of a measured current value outputted to a computer.
[Figure 10(B)] Figure 10(B) shows another example of the measured current value outputted to the computer.
[Figure 11] Figure 11 is a configuration diagram showing an example of a biological polymer characteristic analyzer that measures a current value to identify the position where the biological polymer is trapped.
[Figure 12] Figure 12 is a configuration diagram showing an example of a biological polymer characteristic analyzer that performs optical measurement to identify the position where the biological polymer is trapped.
[Figure 13] Figure 13 is a configuration diagram showing an example of wiring of a thin membrane with pores for trapping and a thin membrane with pores for measurement that identify the position where a biological polymer is trapped based on a measured current value.
[Figure 14] Figure 14 shows an example of the top surface of the thin membrane with pores for trapping that identifies the position where a biological polymer is trapped based on a measured current value.
[Figure 15] Figure 15 is a configuration diagram showing an example of a cross section of a chip with a chamber that separates individual pores.
[Figure 16] Figure 16 is a configuration diagram showing an example of a principle cross section of an in-chamber flow path generator.
[Figure 17(A)] Figure 17(A) is a top-view configuration diagram showing a case where a thin membrane with a signal detection pore is moved for analysis.
[Figure 17(B)] Figure 17(B) is a side-view configuration diagram showing the case where a thin membrane with a signal detection pore is moved for analysis.

### Description of Embodiments

In the present application, a biological polymer means an oligomer and a polymer of biological origin formed of a large number of low-molecule unit structures (monomers). Specifically, a biological polymer in the present application refers, for example, to a DNA, an RNA, and a protein and depends on individual biological bodies. A biological polymer also includes a poly(A), a poly(T), a poly(G), a poly(C), and other artificially synthesized molecules. In the present application, a biological polymer is allowed to pass through a nanopore as a sample whose characteristics are to be examined and is abbreviated to a polymer.

In the present specification, a labeling substance is a substance that is bonded to a biological polymer and labels it. A first effect of a labeling substance is allowing a thin membrane with pores to trap a polymer. A particle having a diameter greater than or equal to the diameter of a nanopore for trapping provided in a thin membrane is used as a labeling substance, which is caught by the nanopore and prevents the polymer from passing through the nanopore or the polymer is trapped in the nanopore. The motion of a particulate labeling substance is arbitrarily controllable by a magnetic field or in accordance with the principle of optical tweezers, whereas the motion of a labeling substance formed of a polymer ion or any other charged particle is controllable by an electric field.

A second effect of a labeling substance is identifying the position where a polymer is trapped. A labeling substance whose material is selected appropriately for observation or detection can be visibly identified with the aid of fluorescence or an optical microscope or electrically measured or otherwise processed for identification. Exemplary materials of a labeling substance having the effect described above may include a fluorescent bead, a substance that absorbs or scatters light, a metallic particle, and a polymer ion having a fluorescence-functional group.

A single substance that simultaneously achieves the two types of effect described above can be used as a labeling substance. A labeling substance can alternatively be formed by combining two substances that independently achieve the two types of effect described above.

Nanopores according to the present application are formed in a thin membrane. A thin membrane with pores according to the present application is classified into two types according to the purpose thereof. A first thin membrane with pores is used to trap a biological polymer bonded to a labeling substance (hereinafter referred to as thin membrane with pores for trapping), and a second thin membrane is used to analyze characteristics of a biological polymer (hereinafter referred to as thin membrane with pores for measurement). The thin membranes with pores described above are used also in two ways as follows: a thin membrane with pores for trapping and a thin membrane with pores for measurement are separate components; and a single thin membrane with pores works not only for trapping but also for measurement.

A thin membrane according to the present application is made of an inorganic material and basically an electrical insulator except the pores. Examples of the thin membrane include SiN or SiO₂. The thin membrane may also contain an insulator made of an organic material or a polymer material. Any of the thin membrane materials may be formed into a monolayer or a multilayer. When a multilayer is formed, the outermost material is an insulator. The thin membrane may have electric wiring for monitoring trapping of a biological polymer or analyzing characteristics of a biological polymer.

A nanopore and a pore in the present application is a nanometer-sized hole provided in the thin membrane described above and passing therethrough from the front surface to the rear surface. Each pore is sized, for example, within the following appropriate ranges depending on an object to be measured: greater than or equal to 1 nm but smaller than or equal to 3 nm, greater than or equal to 3 nm but smaller than or equal to 10 nm, and greater than or equal to 10 nm but smaller than or equal to 50 nm.

Since the diameter of a dsDNA (double-stranded DNA) is 2.6 nm, an appropriate diameter of a pore used to measure a dsDNA ranges from 3 to 5 nm. The diameter of an ssDNA (single-stranded DNA) is 1.5 nm, and an appropriate diameter of a pore for an ssDNA is greater than or equal to 2 nm but smaller than or equal to 3 nm because an ssDNA is likely to form a hairpin structure and hence measured in a modified form in some cases.

Assuming that a substance is appropriately trapped in a nanopore and a barrier is present around the nanopore and separates it from nanopores adjacent thereto, the nanopore can have a diameter ranging from 10 to 50 nm and can be used to measure a dsDNA or a substance having a diameter corresponding to that of a dsDNA.

An opening in the present application refers to the portion of the pore that is visible on each surface of the thin membrane. A polymer, an ion, or any other substance in a solution enters the opening and exits through the opposite opening.

Trap information in the present application refers to information on the position of a biological polymer bonded to a labeling substance trapped by a thin membrane with pores for trapping, whether or not a trapped biological polymer is present, and a trap rate representing the percentage of pores in the thin membrane with pores for trapping that have trapped a biological polymer.

Analysis in the present application refers to analysis of characteristics of a biological polymer. To analyze characteristics of a biological polymer, differences in current characteristic among bases that form the biological polymer are measured.

Two types of current amplifier unit are used in the present application. One of the current amplifier units is a simplified current amplifier unit (first amplifier) that is electrically wired to each nanopore in a thin membrane with pores for trapping and used to check that a biological polymer has been trapped in the nanopores for trapping. The amplifier is so structured that it is compact, simple, and inexpensive to manufacture. The other one of the current amplifier units is a fine current amplifier unit (second amplifier). The second amplifier is electrically wired to all the pores, but a plurality of wiring switchers are so provided between the pores and the second amplifier that only an arbitrary single pore is wired to the second amplifier in single analysis. Since the second amplifier is used to analyze a biological polymer, it is configured to produce a small amount of noise at the time of current measurement and detect and amplify a difference in current magnitude of the order of several pico-amperes.

Blockage current in the present application is produced as follows: A chamber is provided on each side of a thin membrane having pores provided therein. The chambers and pores are filled with an aqueous solution containing an electrolyte (ion), such as KCl, and a voltage is applied between the two chambers. The ion moves and a current (ion current) flows through the pores. The magnitude of the ion current depends on how readily the ion moves, particularly depends on the cross-sectional area of the pores. The magnitude of the ion current increases with the cross-sectional area of the pores. When one of the chambers-is filled with a DNA solution and any of the DNAs passes through a pore, the effective cross-sectional area of the pore decreases, and the magnitude of the ion current decreases accordingly. The phenomenon in which the magnitude of the current decreases when a DNA passes through a pore and blocks the pore is called a blockage current. When chambers and electrodes are disposed on the upper and lower sides of a horizontally placed thin membrane, and a voltage is applied between the electrodes, current flows in the direction perpendicular to the thin membrane. The thus produced current is called a vertical blockage current. In contrast to the vertical blockage current described above, a structure in which electrodes are so buried in a thin membrane that the electrodes 1401 protrude into a pore, and an ion current is allowed to flow through the thin membrane in the horizontal direction. A polymer is then allowed to flow in the pores against the ion current and prevents the ion current from flowing. The resultant decrease in the current is called a horizontal blockage current.

Examples of the present invention will be described below with reference to the drawings.

### (Example 1) Process

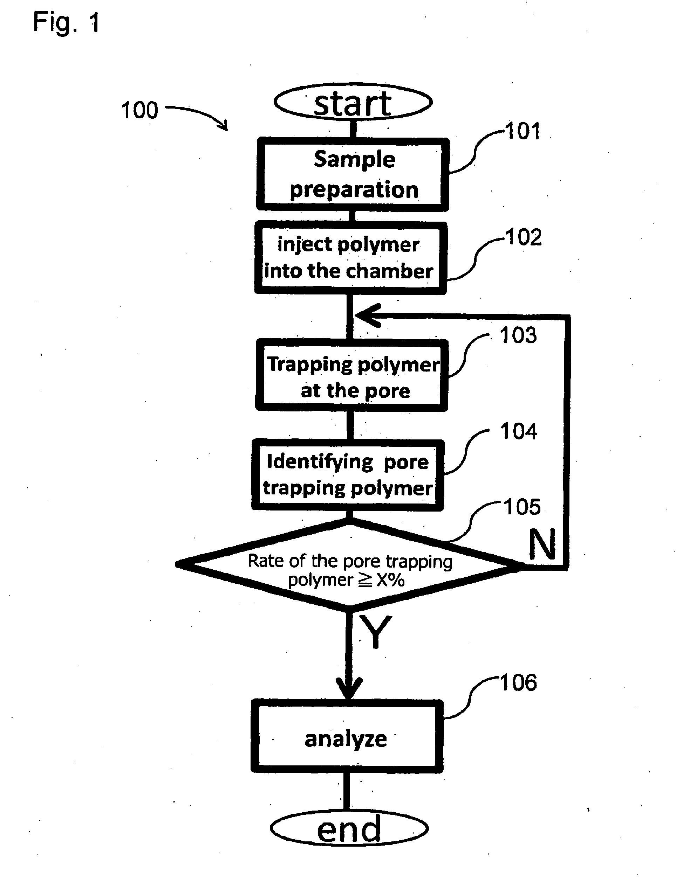

Figure 1 shows an example of the operation of a multi-nanopore analyzer according to the present application. A process 100 of a method for analyzing characteristics of a biological polymer trapped in multiple nanopores with a monitoring unit includes a sample preparation step 101, a biological polymer injection step 102, a biological polymer motion suppression step 103, a trap position identification step 104, a trap rate-based determination step 105, and an analysis step 106, as shown in Figure 1. Each of the steps will be summarized below (will be described later in detail).

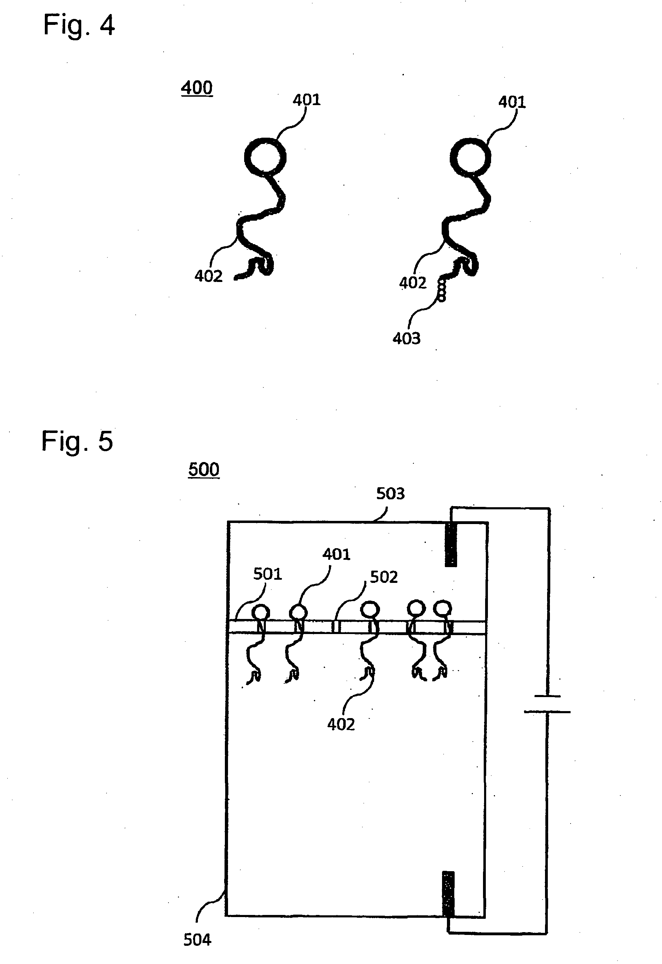

In the sample preparation step 101, a biological polymer whose characteristics are to be analyzed is extracted, for example, from a cell, and a labeling substance having a diameter greater than the diameter of each of the pores provided in the thin membrane and capable of being monitored or detected is bonded to one terminal of the biological polymer to form a biological polymer-labeling substance complex. In the present example, the biological polymer is a DNA, and the labeling substance is a fluorescent labeled particle. Figure 4 is a diagrammatic view showing the form of the biological polymer-labeling substance complex. In the biological polymer injection step 102, the biological polymer-labeling substance complex is injected into one of the chambers. The concentration of the introduced complex is determined in consideration of the volume of the chambers, the size of the biological polymer, and the number of events to be repeated.

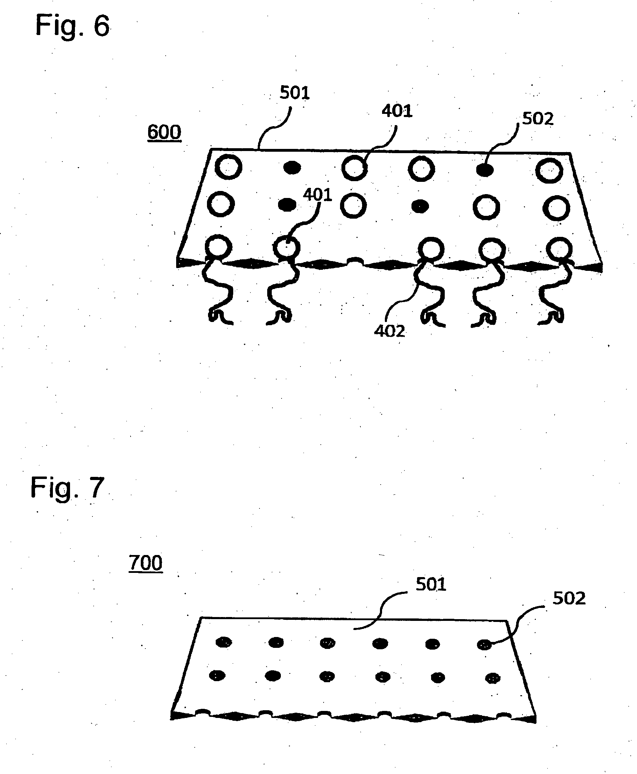

In the biological polymer motion suppression step 103, the biological polymer-labeling substance complex is trapped in pores in the thin membrane. Figure 6 is a diagrammatic view showing how the biological polymer-labeling substance complex is trapped in pores in the thin membrane (600).

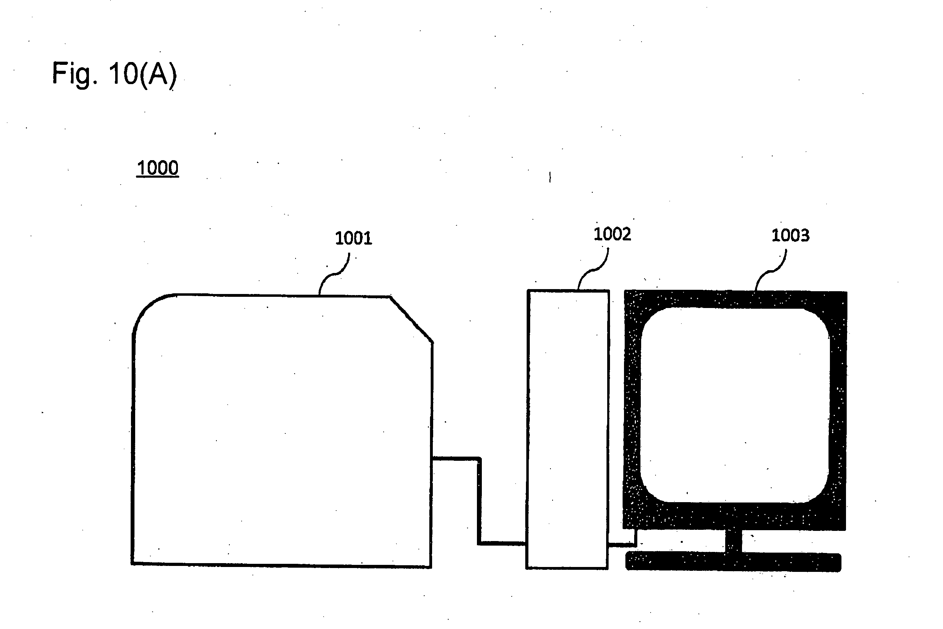

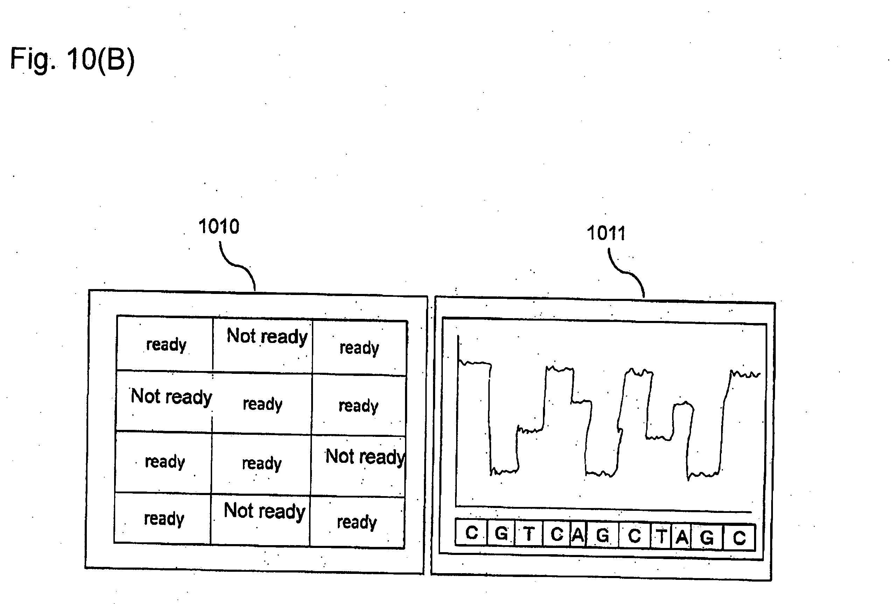

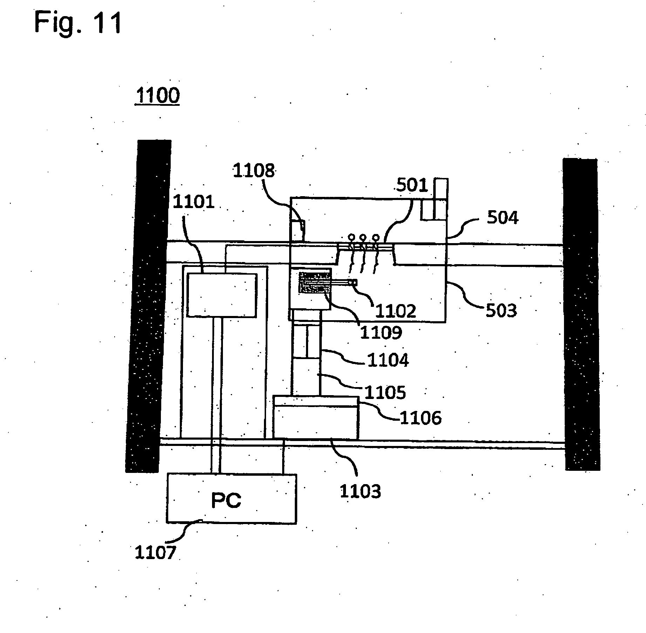

In the trap position identification step 104, trap information acquisition means (also referred to as monitoring means) is used to acquire trap information. In the present example, the monitoring means is a fluorescence microscope, and whether or not the biological polymer is present in each of the nanopores and the coordinates (position) of each of the nanopores in the thin membrane, which are basic elements of the trap information, are identified by acquiring an image of the fluorescent labeled particles, which are the labeling substance. Figure 11 shows an example of the configuration of the monitoring means. The left part of Figure 10(B) shows an example of a result of identified positions where the biological polymer has been trapped.

In the trap rate-based determination step 105, the trap rate is checked and compared with a predetermined value, and the step to be carried out next is determined. The trap rate used herein is defined as the ratio of the number of pores where it is identified that the biological polymer is present in the trap position identification step 104 to the total number of pores in the thin membrane. When the trap rate is greater than or equal to the predetermined value, the following analysis step 106 is carried out, whereas when the trap rate is smaller than the predetermined value, the biological polymer motion suppression step 103 is carried out again.

In the analysis step 106, characteristics of the trapped individual pieces of the biological polymer whose presence has been identified in the trap position identification step 104 are analyzed.

A specific example and other aspects of Example 1 will next be described.

The biological polymer-labeling substance complex prepared in the sample preparation step 101 will be described with reference to Figure 4. Examples of a labeling substance 401 bonded to a biological polymer 402 include a fluorescent labeled particle, a metallic particle, a magnetic bead, a particle formed polymer, and a photo-absorption material. Examples of the fluorescent labeled particle include Cyanine-5 (cy5), Cyanine-3 (cy3), and Indodicarbocyanine-5 (Ic5).

Examples of the particle formed polymer include a latex bead, an acrylic polymer bead, polymethylmethacrylate, and styrene. An example of the photo-absorption material is a black body like material.

Any of the labeling substances is bonded to a polymer, for example, in the form of covalent bond, Van der Waals force, ionic bond, or hydrogen bond. Further, a substance having an amount of charge smaller than that of a DNA or an electrically neutral substance having no charge is preferably used as the labeling substance.

In the sample preparation step 101, to encourage the biological polymer-labeling substance complex to be trapped in the nanopores, a second chemical 403 having a negative charge density greater than that of the biological polymer and having a diameter smaller than that of the nanopores can also be used to label the terminal of the polymer that is not labeled with any labeling substance (non-labeled terminal). Examples of the second chemical 403 having a negative charge density suitable for the purpose of the present application include a polycarboxylate, a polysulphate, and a polyphosphate. A description will be made based on as polyacrylate as an example of the polycarboxylate. A deoxynucleotide, which is a DNA monomer, has a molecular weight of about 325 g/mol and an amount of charge of -1. On the other hand, an acrylic acid, which is a monomer of a polyacrylate, has a molecular weight of about 72 g/mol and an amount of charge of -1. In view of the values described above, labeling a terminal of a DNA with a polyacrylate and applying a positive voltage to the second chamber allows an electrostatic attraction about 4.5 times greater than in the case of a DNA alone per unit molecular weight to act on the DNA and hence the DNA to more forcibly migrate toward the second chamber, whereby the DNA can be more efficiently trapped in a nanopore. Other substances that can be preferably used as the second chemical 403 include a polystyrene sulfonate (molecular weight of monomer: 185), a polyphosphate (molecular weight monomer: 80), and a variety of other ionic polymers. The ratio of the degree of polymerization of an ionic polymer used as the second chemical described above to the degree of polymerization of a sample DNA (ratio between degrees of polymerization) is preferably set at a value ranging from several percent to about 50 percent. The magnitude of the electrostatic force and hence the trap efficiency increase as the ratio between the degrees of polymerization increases, whereas the occupancy rate of the DNA in the sample that passes through a nanopore decreases as the ratio increases. In view of the fact described above, the ratio between the degrees of polymerization is preferably determined in consideration of the trap efficiency and the measurement efficiency.

A specific procedure and operation of the biological polymer motion suppression step 103 will be described below with reference to Figure 5. The biological polymer-labeling substance complex is added to the first chamber 503, and then a force that guides the biological polymer into the second chamber 504 is exerted. As the guiding force, when the biological polymer is, for example, a DNA, a negative voltage having the same polarity as that of negative charge, which a DNA has, is applied to the first chamber, and a positive voltage having the opposite polarity is applied to the second chamber. The positive voltage attracts the negatively charged DNA, which therefore migrates in the direction from the first chamber 503 toward the second chamber 504, specifically, toward a nanopore 502, where the two chambers are in contact with each other. Since the labeling substance, which is less negatively charged than the DNA, is not so encouraged as compared with the DNA to migrate toward the positive voltage, the labeling substance is behind and follows the DNA. As the migration continues, the DNA 402, which is the biological polymer, passes through the nanopore 502 and flows into the second chamber, but the labeling substance 401 at the end of the DNA 402 is trapped at the opening of the chamber because the diameter of the labeling substance 401 is greater than the diameter of the nanopore. As a result, the DNA-labeling substance complex is trapped in the pore formed in the thin membrane. In the following steps, the trapped state is maintained as long as the voltage is applied.

### (Monitoring based on labeling substance - optical method)

In the trap position identification step 104, the biological polymer and the labeling substance form a complex, and the positions of the biological polymer and the labeling substance coincide with the position of the complex. The monitoring means can be selected as appropriate in accordance with the type of label of the labeling substance. For example, when the labeling substance bonded to the biological polymer is a group of fluorescent molecules, a fluorescent bead, or a FRET-based label, the monitoring means can be an optical method (hereinafter referred to as optically monitoring method), preferably a fluorescence microscope in particular. An image captured with the fluorescence microscope is converted by a CCD camera or any other suitable component into image data, which is then processed by a computer. In this way, whether or not the biological polymer is present and the position thereof can be identified.

When a fluorescence microscope is used as the monitoring means, the distance between the nanopore patterns on the thin membrane is set at such a large value that the labeling substance that non-specifically adheres to a portion where no pattern is present is not detected. Further, since the labeling substance trapped in an arbitrary pore radiates fluorescence within a range of about 1 µm, the pores in the thin membrane with pores for trapping are desirably arranged at intervals of at least 2 µm in order to prevent the fluorescence from the labeling substance trapped in the pore from overlapping with the fluorescence from the labeling substance trapped in adjacent pores.

To identify the position of a pore, a guideline that serves as a coordinate reference can be formed on the thin membrane. When the labeling substance is a fluorophor and the monitoring means is a fluorescence microscope, the guideline is drawn, for example, with fluorescent paint on the thin membrane, and a fluorescence image including both the labeling substance and the guideline is captured, whereby the coordinates of the biological polymer on the thin membrane are identified. The guideline may be labeled with the same labeling substance as that for the biological polymer and monitored by using the same monitoring means, or may be labeled with a different labeling substance and monitored by using different monitoring means.

On the other hand, when the labeling substance bonded to the biological polymer is a photo-absorption material or a light scattering material, an optical microscope can be preferably used as the monitoring means used in the optically monitoring method.

In the optically monitoring method, the monitoring means is typically located above the thin membrane where the labeling substance is present, that is, on the side of the first chamber. Alternatively, the monitoring means can be disposed below the thin membrane. In the latter case, it is possible to employ a variation in which a label that can be optically detected, such as a fluorophor having a diameter smaller than that of a pore, is bonded to a non-labeled terminal of the biological polymer in the sample preparation step 101.

### (Monitoring based on thin membrane with nanopores - current method)

In the trap position identification step 104, the monitoring means can alternatively be a current method (hereinafter referred to as current monitoring method). When the current monitoring method is used, a pair of electrodes are provided in the vicinity of each of the pores in the thin membrane, and a voltage is applied horizontally with respect to the surface where the opening is present. A current flowing between the electrodes is measured with a current detection unit. The current detection unit is specifically formed, for example, of a current amplifier, an A/D converter, and a computer. Since the current between the pair of electrodes differs between a case where the biological polymer is present and a case where the biological polymer is not present, whether or not the biological polymer is present in each of the pores can be monitored by measuring the current between each of the pairs of electrodes and comparing the measured current with a preset threshold by using a computer. In the current measurement, a simple current amplifier can be used because only whether or not the biological polymer is present needs to be monitored.

Currents from a plurality of pairs of electrodes can be measured by using a single current detection unit by providing a signal selection unit between the pairs of electrodes and the current detection unit, selecting a specific pair of electrodes based on selection information from the computer, and measuring the current between the selected pair of electrodes. In this case, whether or not the biological polymer is present can be monitored on a single pore position basis by relating the selection information to the positions of the pores in advance. Further, when the current monitoring method is used, since the biological polymer itself provides the second effect of the labeling substance, the labeling substance only needs to have a characteristic necessary for the first effect, that is, a size caught by the pore.

The current monitoring method uses monitoring means formed on the thin membrane, so that the apparatus can be compact and inexpensive. Figure 10(B) conceptually shows a position identification result outputted to the computer.

### (Tunneling current, static capacitance)

As a variation of the current monitoring method, a tunneling current or electrostatic capacitance may be detected as the current described above. In this case, no electrolyte may be added into the chambers in the trap position identification step 104.

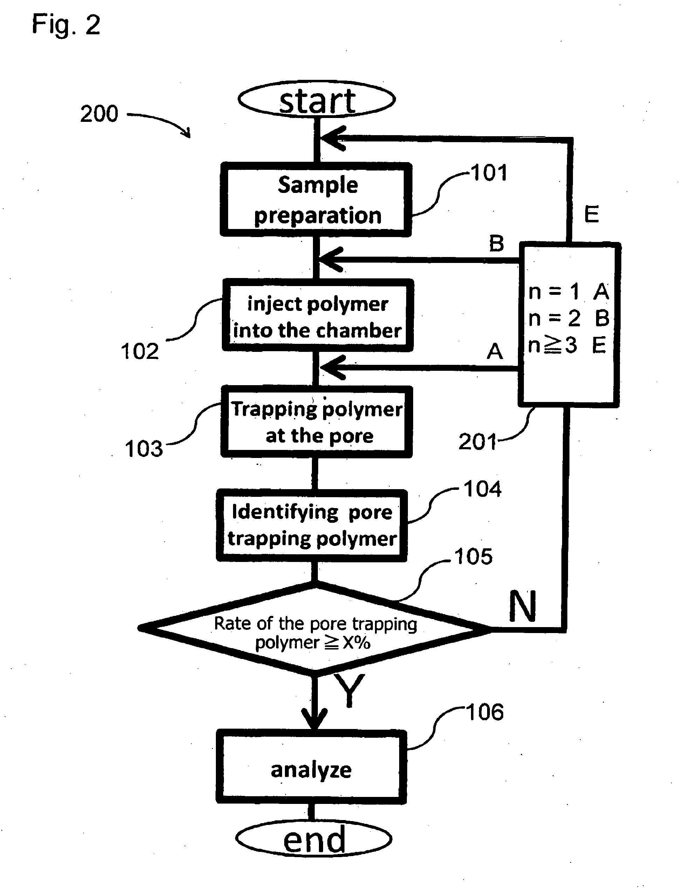

In the trapping rate-based determination step 105, the predetermined value of the trap rate can be set at an arbitrary value in accordance with target process efficiency. When the trap rate is lower than the predetermined value, the biological polymer motion suppression step 103 is carried out again, and any of the following is then carried out: The biological polymer is allowed to be left at rest, the force applied to the nanopores is increased, step 102 is carried out again to increase the amount of biological polymer with which the chamber is filled, or step 101 is carried out again to change the sample, as shown in Figure 2. In some embodiments, a loop formed of the following steps is carried out repeatedly: first changing the magnitude of the applied force and then changing the amount of biological polymer with which the chamber is filled when the changed magnitude of the force provides no change, and the action to be carried out next can be changed in consideration of how many times the loop has been carried out (201). It is preferable to use an analysis method 200 described above.

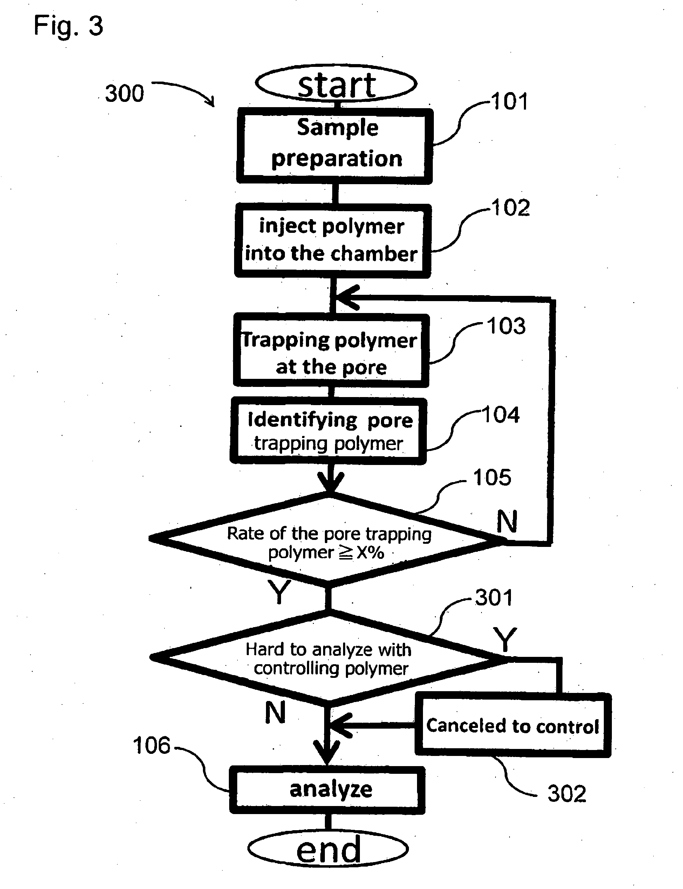

A measurement approach used in the characteristic analysis in the analysis step 106 can, for example, be the blockage current method, the tunneling current method, or the capacitance measuring method. When a single thin membrane works as the thin membrane with pores for trapping and the thin membrane with pores for measurement, any of the methods described above can be employed. When the thin membrane with pores for trapping and the thin membrane with pores for measurement are separate components, any of the horizontal blockage current method, the tunneling current method, and the static capacitance method can be employed. Figure 10(B) shows an example of a measurement result outputted from the measuring apparatus via the computer onto a monitor. When the force applied in the biological polymer motion suppression step 103 prevents the measurement in the characteristic analysis step (301), an applied force canceling step 302 (Figure 3) is carried out. For example, the force prevents the measurement is a case where a biological polymer to which a labeling substance is bonded is trapped in a pore by applying a voltage in the vertical direction and biological molecule characteristics analysis is performed by measuring a blockage current. When the trapping voltage is much higher than a voltage necessary for the blockage current measurement, the measurement cannot be made because the high voltage exceeds the acceptable range of a high-precision amplifier, and the speed at which the biological polymer passes through a pore becomes too fast to read the blockage current in some cases. Further, to read a horizontal blockage current, it is necessary to cancel the applied force otherwise the ion current becomes unstable.

When a biological polymer is analyzed by using nanopores, the motion of the biological polymer can be suppressed in the present example, so that the measurement can be highly precise by setting the measurement period to be sufficiently long and that a measurement error due to Brownian motion can be eliminated. In the present example, multiple pores are provided in a thin membrane and a plurality of pieces of the biological polymer are trapped in the pores formed in the thin membrane in advance for collective measurement, so that the measurement efficiency can be improved. Moreover, in the present example, only the pores in which the biological polymer has been trapped can be measured by identifying the positions where the biological polymer has been trapped in advance, so that no wasted time is spent and hence the operation efficiency and the time efficiency of the measurement apparatus are high. Accordingly, running costs corresponding to the operation period are low and a waiting period to obtain a result is short. That is, the present example provides advantageous effects of high precision, high efficiency, low cost, and quick response.

### (Example 2) Tandem-pore type

An example of the configuration of a multi-nanopore analyzer that includes a trapped sample position monitoring unit and analyzes characteristics of a biological polymer according to the present application will be described with reference to Figure 11. Figure 11 shows part of the biological polymer characteristic analyzer according to the present example. As shown in Figure 11, reference character 501 denotes a thin membrane with pores for trapping a biological polymer. Reference character 503 denotes a chamber filled in with a biological polymer solution. Reference character 1101 denotes a current monitoring unit for identifying a polymer trapped pore position. Reference character 1102 denotes a signal detection pore for measuring a signal specifying characteristics of a biological polymer. Reference characters 1104 to 1106 denote signal detection pore driving units. Reference character 1103 denotes a signal detection unit of tunneling current or static capacitance specifying characteristics of a biological polymer and a driving unit controller. Reference character 1107 denotes a computer.

### (Usage mode)

Measurement operation steps basically follow the procedure in Example 1. There are a number of methods for acquiring biological polymer trap information and performing biological polymer characteristic analysis. Among them, the present example will be presented by using a horizontal blockage current to monitor a biological polymer and the tunneling current method to perform measurement.

### (Thin membrane with pores for trapping biological polymer 501)

The thin membrane with pores for trapping a biological polymer has a plurality of nanopores, and the diameter of each of the nanopores is smaller than the diameter of a labeling substance bonded to the biological polymer. The thin membrane does not necessarily: have a cylindrical shape and may, for example, have a box-like shape. The thickness of the portions where the pores are open is desirably substantially equal to or smaller than the size from the biological polymer to the labeling substance. The thin membrane may be made of any material. When the sequencing is performed based on a blockage current, for example, a silicon oxide membrane or a silicon nitride membrane, but not necessarily, is used. For example, a membrane made of a conductive material, such as p-Si or α-Si, may alternatively be used.

### (Internal structure of thin membrane with pores - wiring)

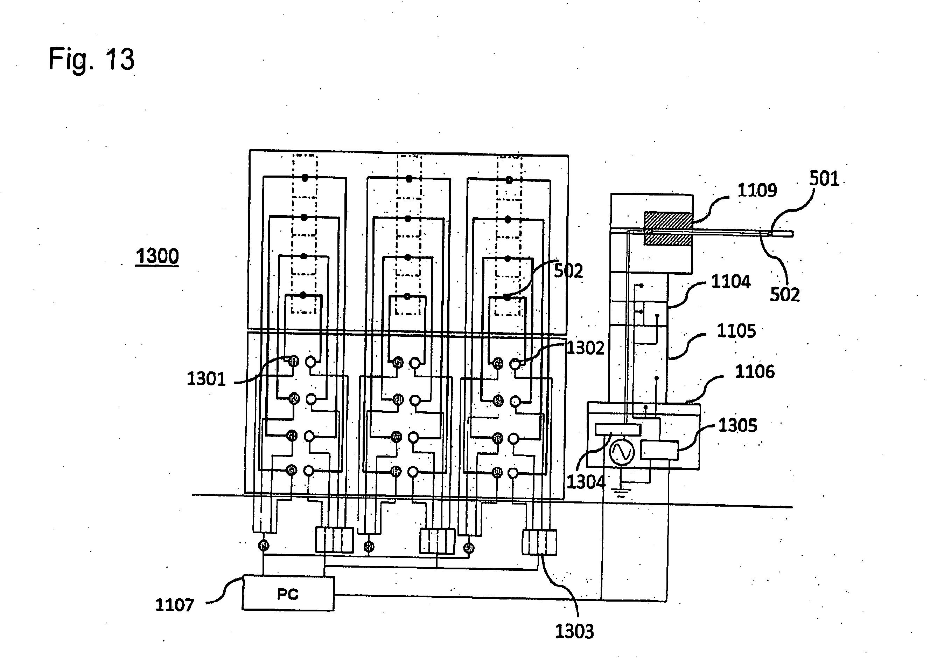

The thin membrane has a layered structure and has a patterned electrode provided therein. The pores are filled with an electrolyte solution, and when a direct current voltage is applied to the electrode, an ion current is produced in the pores. A source of the voltage is built in the current monitoring unit for identifying a polymer trapped pore position 1101. The electrode is wired to all the pores as shown in Figure 13, and each of the wiring lines are connected to a simplified current amplifier unit.

### (Configuration of entire thin membrane with pores)

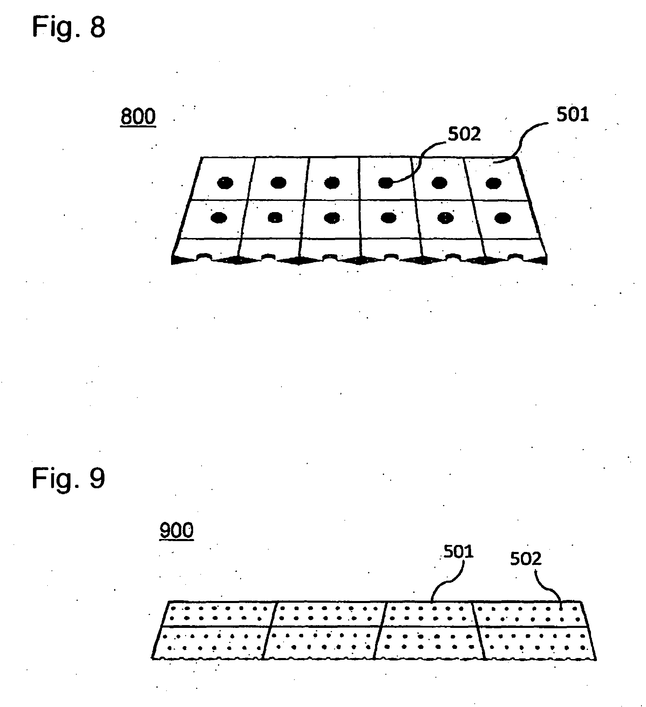

Figure 7 is a diagrammatic view showing the arrangement of the nanopores provided in the thin membrane with pores for trapping a biological polymer. In some embodiments, a plurality of thin membranes each having a single pore are arranged (Figure 8). In other embodiments, a plurality of thin membranes each having multiple pores can be arranged (Figure 9).

When a single substrate having multiple pores (Figure 7) is used, the wiring lines for selecting a pore to be measured can be formed simultaneously with the pores in a single substrate formation process, whereby the number of measurement apparatus manufactured per unit time can be increased.

When a plurality of thin membranes each having a single pore (Figure 8) is used, and the individual pores are independent of each other, an object being measured can be moved toward the measuring apparatus. In this case, pores in which no object is trapped are not measured, and the measuring apparatus does not need to be moved for a long period, whereby the measurement throughput is improved.

When a plurality of thin membranes each having multiple pores (Figure 9) is used, the two advantageous features described above are provided.

### (Option: water flow)

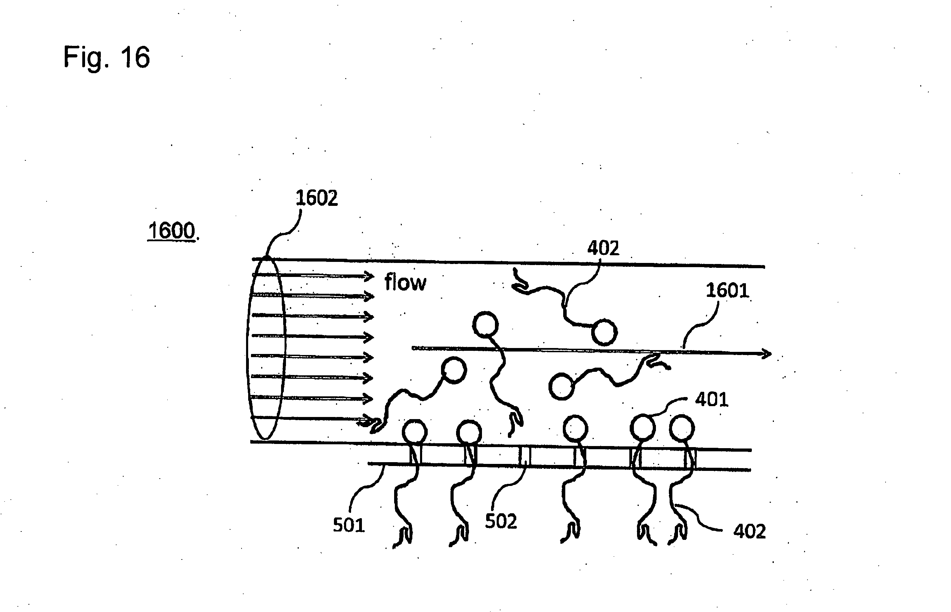

In some embodiments, a portion above the thin membrane with pores in which a biological polymer has been trapped is preferably vibrated in the horizontal direction with respect to the surface of the thin membrane in order to prevent two or more pieces of the biological polymer from being trapped in a single nanopore. The thin membrane with pores for trapping is coupled with a driving unit and radially vibrated by the order of micrometers. The rotation speed preferably ranges from 100 to 3000 rpm. In some embodiments, a transverse flow of liquid is produced in a portion above the nanopores provided in the thin membrane with pores for trapping (enlarged view of Figure 16). The transverse flow can wash off the labeling substance having electrostatically adhered to the pore-less surface of the thin membrane.

The configuration shown in Figure 16 can be used by introducing a water flow generator 1108 in the chamber shown in Figure 11. In this configuration, the intensity of the water flow is desirably so selected that a water flow-induced force that drives the labeling substance is smaller than the voltage-induced force that drives the labeling substance. In some embodiments, the vibration and the water flow described above are preferably combined.

### (Chamber filled in with biological polymer solution 503)

The chamber 503 contains a biological polymer bonded to a labeling substance and an electrolyte solution. The electrolyte solution typically contains KCl and other substances. In some embodiments, NaCl, MgCl₂, and other compounds or a combination thereof may be used. The chamber is made of a material that prevents the solution with which the chamber is filled from leaking out thereof. Further, the chamber is strong enough to hold the solution. A shock-absorbing member 1109 made of a material that absorbs vibration transmitted through a chamber outer wall is disposed in the chamber and sandwiched between the thin membrane and the outer wall. The shock-absorbing member 1109 can be made of polydimethylsiloxane (PDMS) or any other material that absorbs vibration.

### (Current monitoring unit for identifying polymer trapped pore position 1101)

The current monitoring unit for identifying a polymer trapped pore position 1101 is means for identifying a trap position, and an electric source that produces a current flowing through the thin membrane with pores for trapping and simplified current amplifier units 1302 are built in the current monitoring unit 1101. The voltage produced by the electric source can be set by a voltage controller. The thus set voltage controls the trap rate. The simplified current amplifier units 1302 are further connected to an external A/D converter 1303, which is further connected to a computer 1107. Data outputted from the A/D converter 1303 are displayed on a monitor associated with the computer 1107. The positions of the nanopores are related to the output data in advance, and when a nanopore having trapped the biological polymer and provides a change in current greater than a threshold, the nanopore is determined to have trapped the biological polymer.

### (Apparatus configuration used in optical measurement)

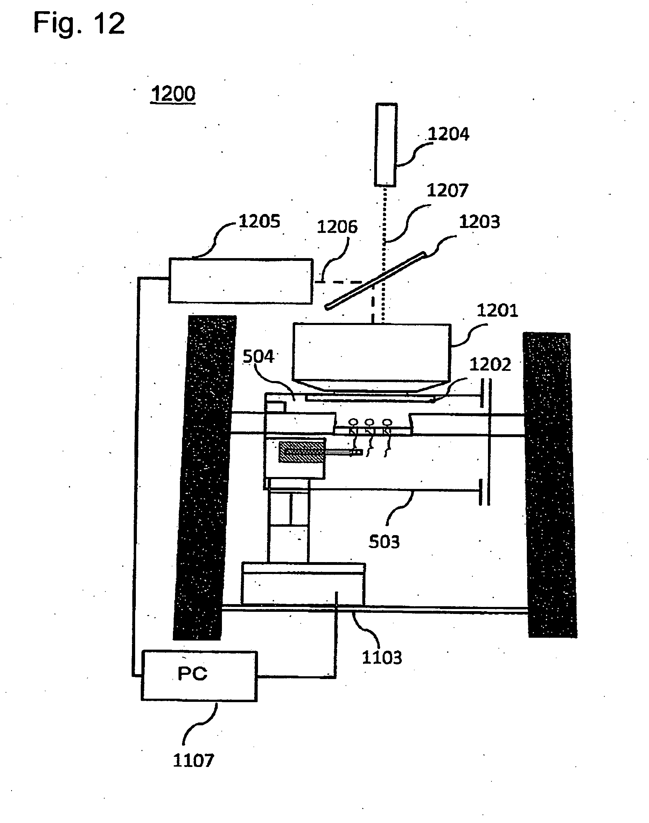

In some embodiments, an optical measurement method can be used as the means for identifying a trap position. Figure 12 shows an example of an apparatus configuration used with an optical measurement method. An observation window 1202 is provided in an upper portion of the chamber, and an objective lens 1201 is provided outside the observation window 1202. A laser 1207 emitted from a laser source 1204 and fluorescence 1206 from a sample are spectroscopically separated at a dichroic mirror 1203. The fluorescence passes through an amplifier unit and a detector 1205 and recorded in the computer 1107.

(Signal detection pore 1102 for measuring signal specifying characteristics of biological polymer)

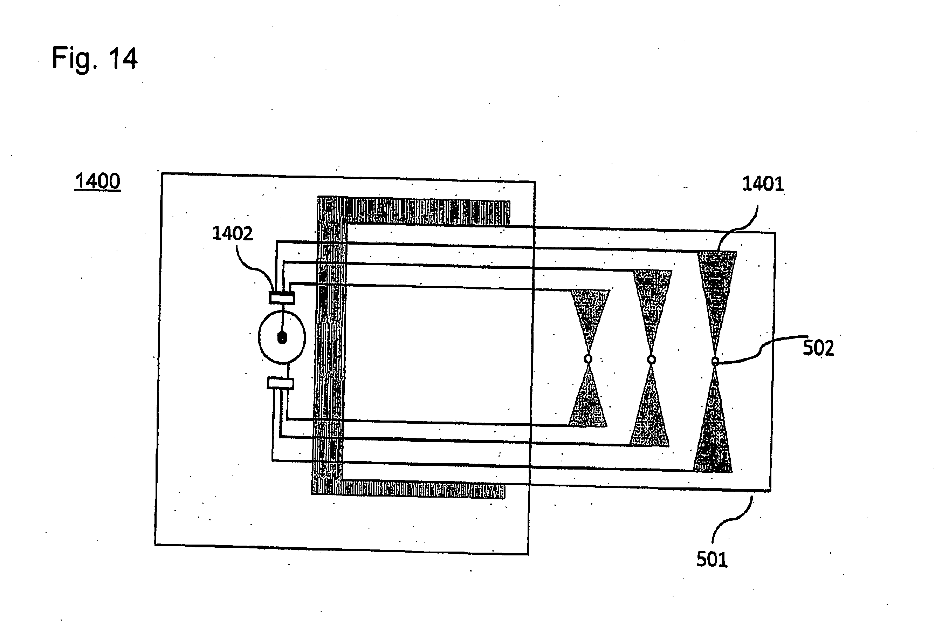

The signal detection pore 1102 for measuring a signal specifying characteristics of a biological polymer measures a tunneling current. Figure 14 is a top view of the thin membrane with pores for measurement, which is connected to the driving units (1104 to 1106), a current monitor 1304, and a driving unit controller 1305, as shown in Figure 13.

A thin membrane for measurement 501 has nanopores 502, and electrodes 1401 are wired to the nanopores. Ends of electrodes on the side facing away from the nanopores pass through a member that fixes the thin membrane and are connected to cables connected to the driving units.

### (Size of pores open through thin membrane/thin member material)

Since the pores formed through a thin membrane with pores for measurement are pores for measuring the base sequence of a polymer, a nanofabricated electrode is buried in the membrane around each of the nanopores (Figure 13). The thin membrane is made primarily of Si, SiN, or SiO₂. To prevent K⁺ and Na⁺ contained in the solution from permeating the thin membrane, SiN is preferably used and SiO₂ is further preferably coated to maintain hydrophilicity. The diameter of the nanopores for measurement needs to be smaller than or equal to 10 nm. Setting the diameter of the nanopores at a value smaller than or equal to 10 nm also advantageously contributes to suppression of Brownian motion of the polymer. The diameter of the pores for measurement is preferably about several nanometers. Since the cross-sectional area of a dsDNA is about 2.5 nm, the diameter of the pores is preferably set to be equal thereto, and it is particularly preferable to set the pore diameter to be slightly greater than 2.5 nm but slightly smaller than two dsDNAs, that is, smaller than 5 nm.

### (Current signal detection unit 1103)

The thin membrane with pores for measurement is wired as shown in Figure 13 and electrically connected to the current signal detection unit having reference numeral 1304, as in the thin membrane with pores for trapping.

The driving units are configured as indicated by 1104 to 1106 in Figure 13, and each of which includes coarse-motion units 1105 and 1106 and a micro-motion unit 1104 for the x, y, and Z directions and the driving unit controller 1305. The operation direction of each of the driving units is determined by the driving unit controller 1305.

The controller 1305 is disposed below the driving units, receives an instruction sent from an executable file in the computer 1107, and controls the travels of the micro-motion unit 1104, the Z-axis coarse-motion unit 1105, and the XY-axis coarse-motion unit 1106.

The driving units 1104 to 1106, which move the thin membrane with a signal detection pore under the nanopores, stops the thin membrane below a nanopore having trapped the biological polymer under the control of the controller 1305, where the characteristics of the biological polymer trapped in the nanopore are analyzed.

In some embodiments, biological polymer trap information is sent to the computer 1107 whenever necessary, and the driving units 1104 to 1106 move the thin membrane with a signal detection pore to the position below another nanopore having trapped the biological polymer whenever necessary, where the characteristics of the trapped biological polymer are analyzed.

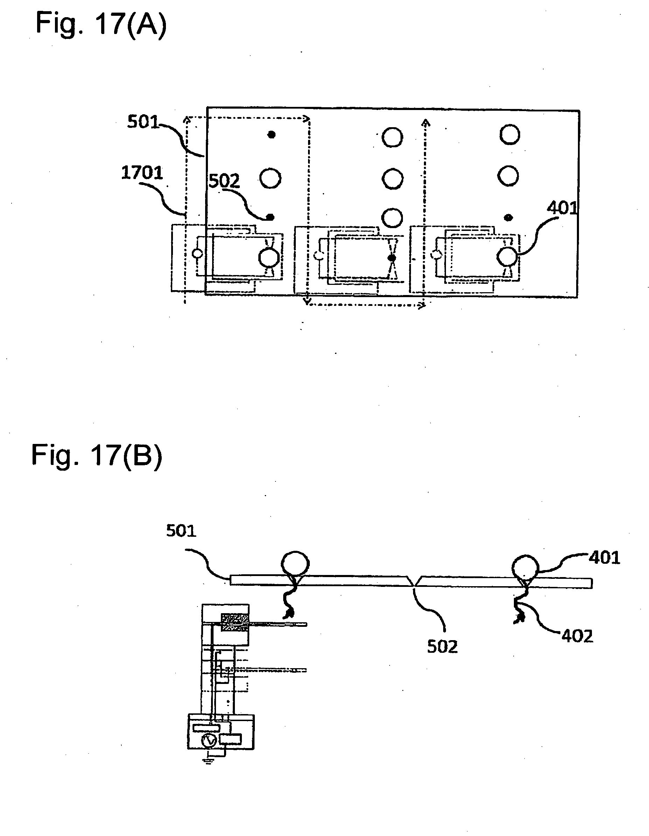

Figure 17(A) shows an exemplary embodiment. In Figure 17(A), a first thin membrane 501, a pore 502 formed therein, and a biological molecule 400 trapped in the pore 502 are drawn, and the path 1701 along which a second pore moves and driving units connected to a second thin membrane are drawn with dotted lines. Figure 17(B) shows the first thin membrane, the biological polymer trapped by the first thin membrane, the second thin membrane, and the driving units viewed in the horizontal direction. The dotted lines represent the path along which the second thin membrane moves. When the driving units are moved to a position below the trapped biological polymer, the z-direction driving unit and the micro-motion unit move the thin membrane, and the biological polymer starts being analyzed.

In some embodiments, biological polymer trap information is sent to the computer 1107 whenever necessary, and the driving units 1104 to 1106 move the second thin membrane to the position below a nanopore having trapped the biological polymer whenever necessary along a path that is not limited to the path shown in Figure 17, where the characteristics of the biological polymer are analyzed.

The micro-motion unit 1104 is an element capable of moving an object in the order of nanometers and can be formed of a piezoelectric device or made of any suitable material. An initial position and the positions immediately below the pores are specified in advance and, the position of the object is controlled in the order of nanometers. Further, each of the coarse-motion units 1105 and 1106 is an element capable of moving an object in the order of micrometers.

The present apparatus can be disposed in any position relative to the position of a thin membrane with pores for trapping. In the examples of the present invention, piezoelectric devices can be used. The apparatus may be integrated with the driving unit controller described above.

Using the measurement means described in the present example to trap a plurality of DNAs of the same type in multiple pores and analyze the DNAs individually allows a result rich in information to be obtained by performing measurement independently and repeatedly many times, that is, in a highly multiple manner. A highly precise result can be obtained by statistically processing the result.

### (Example 3) Non-tandem apparatus configuration

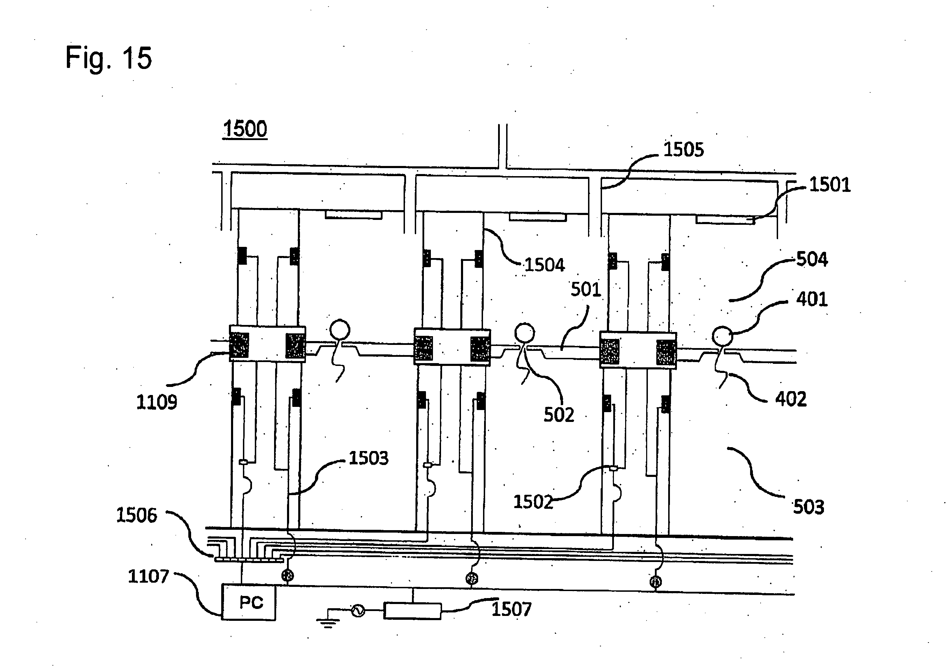

An example of the configuration of a multi-nanopore analyzer that includes a trapped sample position monitoring unit and analyzes characteristics of a biological polymer according to the present application will be described with reference to Figure 15. Figure 15 shows part of the biological polymer characteristic analyzer according to the present example, which includes a thin membrane 501 with pores for trapping a biological polymer and pores for measurement, a biological polymer lifting force generator 1501, a chamber 503 filled in with a biological polymers solution, a current monitoring unit 1502 for identifying a polymer trapped pore position, and a signal detection unit 1503 of blockage current specifying characteristics of a biological polymer, as shown in Figure 15. A method for operating the analyzer and the configuration of the analyzer will be summarized below. The thin membrane 501 with pores for measurement, the biological polymer lifting force generator 1501, the chamber 503 filled in with a biological polymer solution, and the current monitoring unit 1502 for identifying a polymer trapped pore position are integrated to form a single chip.

### (Usage example)

A method for analyzing a biological polymer is basically the same as that in Example 1, but acquisition of biological polymer trap information and analysis of biological polymer characteristics are performed by using the blockage current method in the present example.

### (Detailed description of apparatus configuration)

The thin membrane 501 with pores for trapping a biological polymer and pores for measurement has multiple nanopores, and the size of the pores is 10 nm or smaller to perform analysis based on the blockage current method. The material of the thin membrane 501 is the same as that of the thin membrane with pores for measurement described above (Example 2).

The chamber 503 filled in with the biological polymer solution has a structure including a plurality of barriers that partition the solution around the individual pores. A shock-absorbing member 1109 is provided along the interface between each of the barriers and the thin membrane to absorb vibration from the barrier. In some embodiments, a plurality of thin membranes are connected to a plurality of barriers. In this configuration, the connection is made via the shock-absorbing members 1109 to absorb vibration from the barriers. Representative examples of the shock-absorbing material include PDMS (polydimethylsiloxane) and a silicon rubber.

The biological polymer solution, an electrolyte solution, a buffer, and other solution are allowed to flow through tubes, such as sample filling paths 1505, disposed next to biological polymer lifters and above the chamber.

Two types of wiring for blockage current measurement are provided in the chamber.

### (Wiring 1)

One of the two types of wiring is connected to the individual simplified amplifier units 1502 in order to monitor whether the biological polymer is trapped. The simplified amplifier units are connected to a driving unit and an A/D converter 1506 external to the thin membrane. The A/D converter 1506 is further connected to the computer 1107. In the computer, the positions of the pores are related to information acquired from the converter in advance, and a pore showing a value greater than or equal to a threshold is determined to have trapped the biological polymer. Figure 10(B) shows the PC screen displaying such information (1010). The trap rate of the pores can be acquired from the information.

### (Wiring 2)

The other one of the two types of wiring is connected to a high-precision amplifier unit 1507 built in a signal detection unit 1503 of blockage current specifying characteristics of a biological polymer, which refers to the trap information described above and measures an arbitrary pore selected from the pores by using a switch capable of being electrically connected to any of the pores.

The biological polymer lifting force generator 1501 is positioned in an upper portion of the chamber.

The biological polymer lifting force generator 1501 includes a material having antipolarity and provided in an upper portion of the chamber, and when a substance that labels the biological polymer is a magnetic bead, the amount of blockage current is monitored while the biological polymer is slowly lifted by gradually increasing the biological polymer lifting force. In some embodiments, the labeling substance may have positive charge. In this case, the base sequence is measured by applying a voltage in such a way that the material described above becomes the negative electrode and slowly incrementing the applied voltage.

In some embodiments, a DNA winding-type lifter may alternatively be used. The winder is fixed to a portion around each of the pores and has one end connected to a DNA. The winder rotates and slowly winds a biological polymer in accordance with a reading speed.

During this process, the DNA does not exit from the pore due to a voltage gradient. In some embodiments, a protein or a polymer having long-chain negative charge is trapped at the terminal facing away from the terminal connected to the DNA winder, whereby the DNA does not exit from the pore.

The current monitoring unit 1502 for identifying a polymer trapped pore position and the signal detection unit1503 of blockage current specifying characteristics of a biological polymer are substantially the same as those in Example 2. The two components can be arranged in any way with respect to the other components. In the present example, the simplified amplifier units in the current monitoring unit 1502 for identifying a polymer trapped pore position are buried in the respective barriers 1504, which separate the pores formed in the thin membrane with pores for trapping from each other. The signal detection unit 1503 of specifying characteristics of a biological polymer is disposed outside the thin membrane and connected to electrodes wired to the pores. Further, any type of material, element, and other components can be used.

### Reference Signs List

100 process of method for analyzing characteristics of biological polymer trapped in multiple pores with monitoring unit
101 sample preparation step
102 biological polymer injection step
103 biological polymer motion suppression step
104 trap position identification step
105 trap rate-based determination step
106 analysis step
200 process of method for analyzing characteristics of biological polymer trapped in multiple pores with monitoring unit
201 retry step determination step
300 process of method for analyzing characteristics of biological polymer trapped in multiple pores with monitoring unit
301 determination of whether or not force applied to suppress motion of biological polymer prevents measurement
302 cancel step
400 biological polymer bonded to labeling substance
401 labeling substance
402 biological polymer
403 second chemical
500 biological polymer to which labeling substance is bonded and which is trapped by thin membrane with pores
501 thin membrane
502 nanopore
503 first chamber
504 second chamber
600 biological polymer trapped by thin membrane with pores
700 thin membrane with multiple pores
800 multiple thin membranes each having single nanopore
900 multiple thin membranes each having multiple nanopores
1000 example of data measured when trap information is acquired outputted to computer and example of analyzed characteristics outputted to computer
1100 example of trap information acquisition unit that acquires trap information from current
1101 current monitoring unit for identifying polymer trapped pore position
1102 signal detection pore for measuring signal specifying characteristics of biological polymer
1103 signal detection unit of current specifying characteristics of biological polymer and a driving unit controller
1104 micro-motion unit
1105 z-axis coarse motion unit
1106 xy-axis coarse motion unit
1107 computer
1108 water flow generator
1109 shock-absorbing member
1200 example of trap information acquisition unit that acquires trap information from optical system
1201 objective lens
1202 observation window
1203 dichroic mirror
1204 laser source
1205 detector
1206 fluorescence
1207 laser beam
1300 partial enlarged view of trap information acquisition unit that acquires trap information from current
1301 switch
1302 simplified current amplifier unit
1303 A/D converter
1304 current signal detection unit of specifying characteristics of biological polymer
1305 driving unit controller
1400 top view of thin membrane with pores for measurement
1500 blockage current-based characteristic analysis chip
1501 biological polymer lifting force generator
1502 current monitoring unit for identifying polymer trapped pore position
1503 signal detection unit of current specifying characteristics of biological polymer
1504 barrier
1505 sample filling path
1506 A/D converter
1507 high-precision amplifier unit
1600 water flow generation
1601 water flow
1602 water flow generator
1700 positional relationship among first thin membrane, biological polymer trapped in first pore, and second thin membrane and path of second thin membrane in top view of apparatus
1701 second pore and example of position to which driving unit connected to second pore is moved
1710 positional relationship between biological polymer trapped in first pore and second thin membrane in side view of apparatus

## Claims

1. A biopolymer analysis method using a first thin membrane with multiple nanopores, the method comprising:
a trap step (103) of trapping a plurality of pieces of a biological polymer in the multiple nanopores;
a trap information acquisition step (104) of acquiring trap information by detecting whether or not a piece of the biological polymer has been trapped in each of the multiple nanopores and recording a result of the detection in the form of the trap information being true or false;
a trap rate comparison step (105) of comparing a trap rate that is the ratio of nanopores in which the plurality of pieces of the biological polymer have been trapped to the total number of the multiple nanopores with a preset threshold and, if the trap rate is not greater than the threshold, carrying out again the trap step (103); and
an analysis step (106) of analyzing characteristics of the biological polymer trapped in a nanopore where the trap information is true.

2. The biopolymer analysis method according to claim 1,
wherein the trap step (103) includes applying an electromagnetic field for trapping the plurality of pieces of the biological polymer, and
the method further comprises the step of canceling (302) the electromagnetic field before the analysis step.

3. The biopolymer analysis method according to claim 1,
wherein the plurality of pieces of the biological polymer include a nucleic acid, and
the analysis step (106) includes analyzing the sequence of bases that form the nucleic acid.

4. The biopolymer analysis method according to claim 1,
wherein a second labeling substance having a diameter greater than the diameter of at least one of the multiple nanopores is bonded to one terminal of at least one of the plurality of pieces of the biological polymer, and
in the trap step (103), the second labeling substance is caught by the nanopores so that the biological polymer is trapped in the nanopores.

5. The biopolymer analysis method according to claim 1,
wherein the biological polymer is further added when the trap rate is not greater than the threshold.

6. The biopolymer analysis method according to claim 1,
wherein the trap information includes the position of a trapped piece of the biological polymer.

7. The biopolymer analysis method according to claim 6,
wherein the trap information acquisition step (104) includes acquiring trap information on the multiple nanopores by amplifying a plurality of signals from electrodes provided at the nanopores with amplifiers connected to the electrodes, converting the plurality of amplified signals into digital signals with an A/D converter connected to the amplifiers, grabbing the digital signals into a computer connected to the A/C converter, and using the computer to relate the digital signals to information on the arrangement of the nanopores.

8. The biopolymer analysis method according to claim 6,
wherein the trap information acquisition step (104) includes capturing an image that serves as an index of coordinates on the first thin membrane in advance, further capturing an image of trapped pieces of a labeling substance and acquiring information on light in-tensity from the multiple nanopores to determine the positions of the trapped pieces of the labeling substance, converting the positions into values readable by a computer connected to the first thin membrane via a current monitoring unit, and checking the trap information with the computer.

9. A biopolymer analyzer comprising:
a first thin membrane (501; 700) with multiple nanopores (502) for trapping a biological polymer (402);
trap information acquisition means adapted for acquiring trap information by detecting whether or not a piece of the biological polymer (402) has been trapped in each of the multiple nanopores on the first thin membrane and recording a result of the detection in the form of the trap information being true or false; and
biological polymer characteristic analysis means adapted for analyzing the biological polymer based on the trap information;
wherein trap information acquisition means are adapted to compare a trap rate that is the ratio of nanopores in which the plurality of pieces of the biological polymer have been trapped to the total number of the multiple nanopores with a preset threshold and, if the trap rate is not greater than the threshold, to continue trapping the biological polymer in the multiple nanopores of the first thin membrane; and
wherein the biological polymer characteristic analysis means are adapted to analyze characteristics of the biological polymer trapped in a nanopore where the trap information is true.

10. The biopolymer analyzer according to claim 9,
wherein the first thin membrane (501; 700) includes a current monitor (1101).

11. The biopolymer analyzer according to claim 9,
further comprising a second thin membrane with multiple nanopores (502),
wherein each of the multiple nanopores in the second thin membrane includes a current monitor.

12. The biopolymer analyzer according to claim 9,
wherein the trap information acquisition means includes the first thin membrane (501; 700), a first amplifier (1302) provided external to the first thin membrane, and a pair of electrodes facing each other in each of the multiple nanopores (502) in the first thin membrane, and
wiring from the pairs of electrodes is connectable to a component external to the first thin membrane.

13. The biopolymer analyzer according to claim 12,
wherein the trap information acquisition means further includes an A/D converter (1303) connected to the first amplifier (1302) and a computer (1107) connected to the A/D converter, and
the computer is adapted to acquire an output from the first amplifier via the A/D converter, to relate the output to information on the positions of the multiple nanopores, and to record the relationship as the trap information.

## Patentansprüche

1. Biopolymer-Analyseverfahren unter Verwendung einer ersten dünnen Membran mit mehreren Nanoporen, wobei das Verfahren umfasst:
einen Einfangschritt (103) zum Einfangen mehrerer Teile eines biologischen Polymers in den mehreren Nanoporen;
einen Einfanginformation-Erfassungsschritt (104), bei dem Einfanginformationen durch Detektieren, ob ein Teil des biologischen Polymers in jeder der mehreren Nanoporen eingefangen worden ist oder nicht, erfasst werden und ein Ergebnis der Detektion in der Form, dass die Einfanginformationen wahr oder falsch sind, aufgezeichnet wird;
einen Einfangrate-Vergleichsschritt (105), bei dem eine Einfangrate, die das Verhältnis von Nanoporen, in denen die mehreren Teile des biologischen Polymers eingefangen worden sind, zur Gesamtanzahl der mehreren Nanoporen ist, mit einem vorgegebenen Schwellenwert verglichen wird und, falls die Einfangrate nicht größer als der Schwellenwert ist, der Einfangschritt (103) erneut ausgeführt wird; und
einen Analyseschritt (106), bei dem charakteristische Eigenschaften des in einer Nanopore eingefangenen biologischen Polymers, wenn die Einfanginformation wahr ist, analysiert werden.

2. Biopolymer-Analyseverfahren nach Anspruch 1,
wobei der Einfangschritt (103) das Anlegen eines elektromagnetischen Feldes zum Einfangen der mehreren Teile des biologischen Polymers beinhaltet und
das Verfahren ferner das Auflösen (302) des elektromagnetischen Feldes als Schritt vor dem Analyseschritt umfasst.

3. Biopolymer-Analyseverfahren nach Anspruch 1,
wobei die mehreren Teile des biologischen Polymers eine Nukleinsäure enthalten und
der Analyseschritt (106) das Analysieren der Sequenz der Basen, die die Nukleinsäure bilden, beinhaltet.

4. Biopolymer-Analyseverfahren nach Anspruch 1,
wobei eine zweite Markierungssubstanz mit einem Durchmesser größer als der Durchmesser mindestens einer der mehreren Nanoporen mit einem Endstück mindestens eines der mehreren Teile des biologischen Polymers verbunden wird und
die zweite Markierungssubstanz im Einfangschritt (103) von den Nanoporen gefangen wird, sodass das biologische Polymer in den Nanoporen eingefangen wird.

5. Biopolymer-Analyseverfahren nach Anspruch 1,
wobei das biologische Polymer ferner hinzugefügt wird, wenn die Einfangrate nicht größer als der Schwellenwert ist.

6. Biopolymer-Analyseverfahren nach Anspruch 1,
wobei die Einfanginformationen die Position eines eingefangenen Teils des biologischen Polymers enthalten.

7. Biopolymer-Analyseverfahren nach Anspruch 6,
wobei der Einfanginformation-Erfassungsschritt (104) das Erfassen von Einfanginformationen für die mehreren Nanoporen durch Verstärken mehrerer Signale von an den Nanoporen vorgesehenen Elektroden mit mit den Elektroden verbundenen Verstärkern, das Umwandeln der mehreren verstärkten Signale in digitale Signale mit einem mit den Verstärkern verbundenen A/D-Wandler, das Einspeisen der digitalen Signale in einen mit dem A/D-Wandler verbundenen Computer und das Verwenden des Computers dazu, die digitalen Signale mit Informationen über die Anordnung der Nanoporen in Beziehung zu setzen, beinhaltet.

8. Biopolymer-Analyseverfahren nach Anspruch 6,
wobei der Einfanginformation-Erfassungsschritt (104) das Aufnehmen eines Bildes, das als ein Koordinatenindex auf der ersten dünnen Membran dient, im Voraus, ferner das Aufnehmen eines Bildes eingefangener Teile einer Markierungssubstanz und das Erfassen von Informationen über Lichtintensität von den mehreren Nanoporen zum Bestimmen der Positionen der eingefangenen Teile der Markierungssubstanz, das Umwandeln der Positionen in Werte, die von einem über eine Stromüberwachungseinheit mit der ersten dünnen Membran verbundenen Computer lesbar sind, und das Überprüfen der Einfanginformationen mit dem Computer beinhaltet.

9. Biopolymer-Analyseeinrichtung, umfassend:
eine erste dünne Membran (501; 700) mit mehreren Nanoporen (502) zum Einfangen eines biologischen Polymers (402);
eine Einfanginformation-Erfassungseinrichtung, die dazu ausgelegt ist, Einfanginformationen durch Detektieren, ob ein Teil des biologischen Polymers (402) in jeder der mehreren Nanoporen auf der ersten dünnen Membran eingefangen worden ist oder nicht, zu erfassen und ein Ergebnis der Detektion in der Form, dass die Einfanginformationen wahr oder falsch sind, aufzuzeichnen;
eine Analyseeinrichtung für charakteristische Eigenschaften eines biologischen Polymers, die dazu ausgelegt ist, das biologische Polymer auf der Grundlage der Einfanginformationen zu analysieren;
wobei die Einfanginformation-Erfassungseinrichtung dazu ausgelegt ist, eine Einfangrate, die das Verhältnis von Nanoporen, in denen die mehreren Teile des biologischen Polymers eingefangen worden sind, zur Gesamtanzahl der mehreren Nanoporen ist, mit einem vorgegebenen Schwellenwert zu vergleichen und, falls die Einfangrate nicht größer als der Schwellenwert ist, das Einfangen des biologischen Polymers in den mehreren Nanoporen auf der ersten dünnen Membran fortzusetzen; und
wobei die Analyseeinrichtung für charakteristische Eigenschaften eines biologischen Polymers dazu ausgelegt ist, charakteristische Eigenschaften des in einer Nanopore eingefangenen biologischen Polymers, wenn die Einfanginformation wahr ist, zu analysieren.

10. Biopolymer-Analyseeinrichtung nach Anspruch 9,
wobei die erste dünnen Membran (501; 700) eine Stromüberwachungseinrichtung (1101) enthält.

11. Biopolymer-Analyseeinrichtung nach Anspruch 9,
ferner umfassend eine zweite dünne Membran mit mehreren Nanoporen (502);
wobei jede der mehreren Nanoporen in der zweiten dünnen Membran eine Stromüberwachungseinrichtung enthält.

12. Biopolymer-Analyseeinrichtung nach Anspruch 9,
wobei die Einfanginformation-Erfassungseinrichtung die erste dünnen Membran (501; 700), einen extern zur ersten dünnen Membran vorgesehenen ersten Verstärker (1302) und ein Paar von Elektroden, die einander in jeder der mehreren Nanoporen (502) in der ersten dünnen Membran zugewandt sind, enthält und
die Verdrahtung der Elektrodenpaare mit einer zur ersten dünnen Membran externen Komponente verbunden werden kann.

13. Biopolymer-Analyseeinrichtung nach Anspruch 12,
wobei die Einfanginformation-Erfassungseinrichtung ferner einen mit dem ersten Verstärker (1302) verbundenen A/D-Wandler (1303) und einen mit dem A/D-Wandler verbundenen Computer (1107) enthält und
der Computer dazu ausgelegt ist, eine Ausgabe vom ersten Verstärker über dem A/D-Wandler zu erfassen, die Ausgabe mit Informationen über die Positionen der mehreren Nanoporen in Beziehung zu setzen und die Beziehung als die Einfanginformationen aufzuzeichnen.

## Revendications

1. Procédé d'analyse de biopolymères utilisant une première membrane mince avec de multiples nanopores, le procédé comportant :
une étape de piégeage (103) consistant à piéger une pluralité de fragments d'un polymère biologique dans les nanopores multiples,
une étape d'acquisition d'informations de piégeage (104) consistant à acquérir une information de piégeage en détectant si oui ou non un fragment du polymère biologique a été piégé dans chacun des multiples nanopores et enregistrer un résultat de la détection sous la forme de l'information de piégeage étant vraie ou fausse,
une étape de comparaison de taux de piégeage (105) consistant à comparer un taux de piégeage qui est le rapport des nanopores dans lesquels la pluralité de fragments du polymère biologique a été piégée sur le nombre total des multiples nanopores, avec un seuil préréglé et si le taux de piégeage n'est pas supérieur au seuil, exécuter de nouveau l'étape de piégeage (103), et
une étape d'analyse (106) consistant à analyser des caractéristiques du polymère biologique piégé dans un nanopore lorsque l'information de piégeage est vraie.

2. Procédé d'analyse de biopolymères selon la revendication 1,
dans lequel l'étape de piégeage (103) inclut l'application d'un champ électromagnétique pour piéger la pluralité de fragments du polymère biologique, et
le procédé comporte en outre l'étape consistant à annuler (302) le champ électromagnétique avant l'étape d'analyse.

3. Procédé d'analyse de biopolymères selon la revendication 1,
dans lequel la pluralité de fragments du polymère biologique inclut un acide nucléique, et
l'étape d'analyse (106) inclut l'analyse de la séquence des bases qui forment l'acide nucléique.

4. Procédé d'analyse de biopolymères selon la revendication 1,
dans lequel une seconde substance de marquage ayant un diamètre supérieur au diamètre d'au moins un des nanopores multiples est liée à une terminaison d'au moins un fragment parmi la pluralité de fragments du polymère biologique, et
à l'étape de piégeage (103), la seconde substance de marquage est capturée par les nanopores de sorte que le polymère biologique est piégé dans les nanopores.

5. Procédé d'analyse de biopolymères selon la revendication 1,
dans lequel le polymère biologique est en outre ajouté lorsque le taux de piégeage n'est pas supérieur au seuil.

6. Procédé d'analyse de biopolymères selon la revendication 1,
dans lequel l'information de piégeage inclut la position d'un fragment piégé du polymère biologique.

7. Procédé d'analyse de biopolymères selon la revendication 6,
dans lequel l'étape d'acquisition d'informations de piégeage (104) inclut l'acquisition d'informations de piégeage sur les nanopores multiples en amplifiant une pluralité de signaux provenant d'électrodes disposées sur les nanopores avec des amplificateurs reliés aux électrodes, la conversion de la pluralité de signaux amplifiés en signaux numériques avec un convertisseur A/D relié aux amplificateurs, la capture des signaux numériques dans un ordinateur relié au convertisseur A/D, et l'utilisation de l'ordinateur pour associer les signaux numériques à des informations concernant la disposition des nanopores.

8. Procédé d'analyse de biopolymères selon la revendication 6,
dans lequel l'étape d'acquisition d'informations de piégeage (104) inclut la capture d'une image qui sert d'index de coordonnées sur la première membrane mince à l'avance, la capture supplémentaire d'une image de fragments piégés d'une substance de marquage et l'acquisition d'informations concernant l'intensité lumineuse provenant des nanopores multiples pour déterminer les positions des fragments piégés de la substance de marquage, la conversion des positions en valeurs lisibles par un ordinateur relié à la première membrane mince via une unité de surveillance d'intensité, et la vérification des informations de piégeage avec l'ordinateur.

9. Analyseur de biopolymères comportant :
une première membrane mince (501 ; 700) avec de multiples nanopores (502) pour piéger un polymère biologique (402),
des moyens d'acquisition d'informations de piégeage adaptés pour acquérir une information de piégeage en détectant si oui ou non un fragment du polymère biologique (402) a été piégé dans chacun des nanopores multiples sur la première membrane mince et l'enregistrement d'un résultat de la détection sous la forme de l'information de piégeage étant vraie ou fausse, et des moyens d'analyse de caractéristiques de polymère biologique adaptés pour analyser le polymère biologique sur la base de l'information de piégeage,
dans lequel les moyens d'acquisition d'informations de piégeage sont adaptés pour comparer un taux de piégeage qui est le rapport des nanopores dans lesquels la pluralité de fragments du polymère biologique ont été piégés sur le nombre total des nanopores multiples, avec un seuil préréglé et si le taux de piégeage n'est pas supérieur au seuil, continuer à piéger le polymère biologique dans les nanopores multiples de la première membrane mince, et
dans lequel les moyens d'analyse de caractéristiques de polymère biologique sont adaptés pour analyser des caractéristiques du polymère biologique piégé dans un nanopore lorsque l'information de piégeage est vraie.

10. Analyseur de biopolymères selon la revendication 9, dans lequel la première membrane mince (501 ; 700) inclut un moniteur d'intensité (1101).

11. Analyseur de biopolymères selon la revendication 9,
comportant en outre une seconde membrane mince avec des nanopores multiples (502),
dans lequel chacun des nanopores multiples dans la seconde membrane mince inclut un moniteur d'intensité.

12. Analyseur de biopolymères selon la revendication 9,
dans lequel les moyens d'acquisition d'informations de piégeage incluent la première membrane mince (501 ; 700), un premier amplificateur (1302) disposé à l'extérieur de la première membrane mince, et une paire d'électrodes dirigées l'une vers l'autre dans chacun des nanopores multiples (502) dans la première membrane mince, et
des fils provenant de la paire d'électrodes peut être raccordé à un composant extérieur à la première membrane mince.

13. Analyseur de biopolymères selon la revendication 12,
dans lequel les moyens d'acquisition d'informations de piégeage incluent en outre un convertisseur A/D (1303) relié au premier amplificateur (1302) et un ordinateur (1107) relié au convertisseur A/D, et l'ordinateur est adapté pour acquérir une sortie provenant du premier amplificateur via le convertisseur A/D, pour associer la sortie à des informations concernant les positions des nanopores multiples, et pour enregistrer la relation en tant qu'information de piégeage.
